# EUROPEAN PATENT SPECIFICATION

(11) **EP 3 116 956 B1**
(45) Date of publication and mention of the grant of the patent: **15.05.2019**
(21) Application number: 15709052.3
(22) Date of filing: 27.02.2015
(51) Int. Cl.: C09C 1/30, C08F 2/10, C08F 8/50, C01B 33/12, C08F 20/14, A61K 8/25, A61Q 19/00, A61K 8/02, B01J 13/04, B01J 13/18, B01J 13/20, C01B 33/18, A61K 9/51

(54) **PROCESS FOR PREPARING RECYCLABLE TEMPLATE HOLLOW PARTICLES USING WATER-BASED SILICA PRECURSORS**
VERFAHREN ZUR HERSTELLUNG VON RECYCELBAREN VORLAGENHOHLPARTIKELN MITHILFE VON WASSERBASIERTEN KIESELSÄUREVORLÄUFERN
PROCEDE DE PREPARATION DE PARTICULES CREUSES DE GABARIT RECYCLABLES UTILISANT DES PRECURSEURS DE SILICE A BASE D'EAU

(30) Priority: 11.03.2014 US 201461950875 P
(43) Date of publication of application: 18.01.2017
(73) Proprietor: The Chemours Company FC, LLC, Wilmington, DE 19899 (US)
(72) Inventor: LASIO, Jelena, Bel Air, Maryland 21015 (US); ROSEN, Brad M., Philadelphia, Pennsylvania 19146 (US); LEE, Hau-Nan, Wilmington, Delaware 19808 (US); WHIPPLE, Devin T., Gilbert, Arizona 85296 (US); WOERNER, Francis J., Bear, Delaware 19701 (US)
(74) Representative: Dannenberger, Oliver Andre
(86) International application number: PCT/US2015/017904
(87) International publication number: WO 2015/138157

(56) References cited:
- WO-A1-2013/174753
- KR-A- 20120 056 337
- US-A1- 2007 036 736

## Description

### BACKGROUND OF THE DISCLOSURE

The present disclosure relates to a process for making hollow particles using a template approach, onto which a shell material is deposited, and the template material removed to generate the hollow particle. More particularly, the disclosure relates to a process in which template material can be recycled.

Nano core/shell particles are submicroscopic colloidal systems composed of a solid or liquid core surrounded by a thin polymer or inorganic shell. This solid or liquid core is removed to form hollow nanospheres. Such core-shell systems may be prepared by deposition of the shell material onto a template particle, wherein the shell material can be either organic, inorganic, or hybrid. The selective removal of the core (template) material without disturbing the shell generates hollow particles.

In many cases where a template approach to hollow particles is used, the methods for removing the core are destructive. Typically, the template material is irreversibly changed, and cannot be used again. The most common ways of removing an organic template particle is calcination, whereupon the core material is burned. Alternatively, in the case of acid-labile metal carbonates (such as CaCO₃, for example), the core material can be dissolved in acid, generating a water-soluble metal salt and CO₂.

KR 2012 0056337 A describes a method for manufacturing hollow silica particles by depositing silica onto an electrolyte complex. WO 2013/174753 A1 describes a method of making hybrid organic-inorganic core-shell nano-particles using colloidal organic particles as a template. US 2007/036736 A1 describes a method for making hollow silica particles wherein a silica-containing compound is deposited as a coating on a template particle.

Therefore, a need exists to generate template-based methods for the preparation of hollow particle in which core materials can be recycled.

### SUMMARY OF THE DISCLOSURE

In the first aspect, the disclosure provides a process for making hollow inorganic particles through a template approach, and allows for recycling of the template material. The process for preparing the hollow particles comprises:
(a) providing a recyclable template particle in an aqueous dispersion, wherein the recyclable template particle is prepared from an organic monomer;
(b) coating the recyclable template particle with a water-based silica precursor;
(c) maintaining the pH at about 2 to about 10 to form core/shell particles comprising a silica treatment on the recyclable template particle;
(d) removing the core/shell particles;
(e) removing the recyclable template particle from the core/shell particles by depolymerization through thermal depolymerization, acid hydrolysis or base hydrolysis to form a hollow silica particle; and
(f) recycling the organic monomer after depolymerization of the recyclable template particle.

The recyclable template particle may be a solid particle or a hollow particle. If removed by thermal depolymerization, it is typically heated at temperatures of about 60 °C to about 500 °C.

### BRIEF DESCRIPTION OF THE DRAWINGS

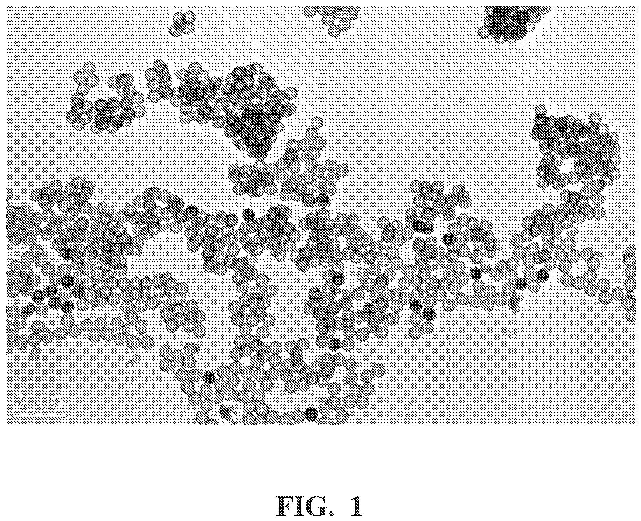

Figure 1 shows the TEM image of PMMA/silica core/shell particles obtained through water-based silica deposition onto PMMA template particles, as described in Example 2.

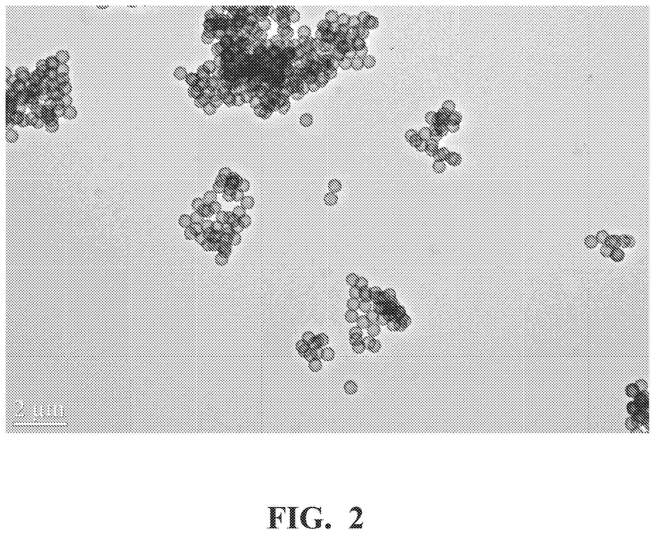

Figure 2 shows the TEM image of hollow silica particles obtained from calcination of PMMA/silica core/shell particles, as described in Example 3.

### DETAILED DESCRIPTION OF THE DISCLOSURE

In this disclosure "comprising" is to be interpreted as specifying the presence of the stated features, integers, steps, or components as referred to, but does not preclude the presence or addition of one or more features, integers, steps, or components, or groups thereof. Additionally, the term "comprising" is intended to include examples encompassed by the terms "consisting essentially of" and "consisting of." Similarly, the term "consisting essentially of" is intended to include examples encompassed by the term "consisting of."

In this disclosure, when an amount, concentration, or other value or parameter is given as either a range, typical range, or a list of upper typical values and lower typical values, this is to be understood as specifically disclosing all ranges formed from any pair of any upper range limit or typical value and any lower range limit or typical value, regardless of whether ranges are separately disclosed. Where a range of numerical values is recited herein, unless otherwise stated, the range is intended to include the endpoints thereof, and all integers and fractions within the range. It is not intended that the scope of the disclosure be limited to the specific values recited when defining a range.

In this disclosure, terms in the singular and the singular forms "a," "an," and "the," for example, include plural referents unless the content clearly dictates otherwise. Thus, for example, reference to "hollow particle", "the hollow particle", or "a hollow particle" also includes a plurality of hollow particles.

This disclosure provides a process for preparing the hollow inorganic particles, through intermediacy of template particles, onto which the shell material is deposited, to generate core/shell particles. The core material is removed to generate hollow particles. This process details the method for generating hollow particles through non-destructive core removal, thereby allowing core material recycling.

The particles described herein are between about a 100 to about 900nm in size, more typically between about 150 and about 800nm, and still more typically between about 230 and about 700nm. The disclosure provides the process for making hollow particles through a template approach in which template material is isolable and recyclable.

The process for preparing the hollow particles comprises:
(a) providing a recyclable template particle in an aqueous dispersion, wherein the recyclable template particle is prepared from an organic monomer;
(b) coating the recyclable template particle with a water-based silica precursor;
(c) maintaining the pH at about 2 to about 10 to form core/shell particles comprising a silica treatment on the recyclable template particle;
(d) removing the core/shell particles; and
(e) removing the recyclable template particle from the core/shell particles to form a hollow silica particle.

The recyclable template particle, that may be a solid particle or a hollow particle, is removed by thermal depolymerization, typically by heating at temperatures of about 60 °C to about 500 °C, or by acid or base.

The recyclable template particle or core is prepared using typically an organic monomer which is polymerized to generate template particles. Some monomes for the template include styrene, methyl methacrylate, α-methylstyrene, lactic acid, or formaldehyde, more typically methyl methacrylate, lactic acid, or α-methylstyrene, and still more typically methyl methacrylate or α-methylstyrene . Similarly, a group of two monomers can be chosen for a copolymerization, such as a variety of diacids and dialcohols for polyester polymers (like polyethylene terephthalate, PET), diacids and diamides for various polyamides (like Nylon 6,6, or other Nylons), etc. The monomers are present in the amount of about 1 to about 60wt%, more typically about 2 to about 50wt%, still more typically about 5 to about 40wt%, based on the total weight of the components used in the preparation of the recyclable template particle. Typically, the particle size of the template is tunable, and the particle size distribution of the template particles achieved is narrow, which is advantageous. For example, preparation of the recyclable template particle or core by emulsion polymerization is achieved by emulsification of the water-insoluble monomer or a monomer mixture in water, and polymerized using radical or photopolymerization conditions. Radical initiators such as potassium- or ammonium persulfate, and 2,2-azobis(2-methylpropionamidine) hydrochloride (AlBA) can be used, more typically AlBA. Surfactant can also typically be used. Some examples of suitable surfactants include sodium dodecylsulfate (SDS), cetyltrimethylammonium bromide (CTAB), poly-(vinylpyrrolidinone) PVP, etc. In some cases, it might be advantageous to use copolymers in order to introduce charge on the surface of the particle, like for example vinyltimethylammonium chloride benzene, 2-(methacryloxy)-ethyltrimethylammonium chloride, etc. In cases where silica is deposited onto the template surface, it might be beneficial to use a copolymer with a silyl group, to promote the silica deposition on the particle surface like for instance 3-(trimethoxysilyl)propylmethacrylate, or other silyl-containing monomers. In some cases, it might be preferable to use comonomers that can crosslink two growing polymer chains, thereby strengthening the template particle-some of those materials include divinylbenzene or ethylene glycol dimethacrylate. In order to perform the polymerization, the reaction temperature is kept between about 0 and about 100 °C, more typically about 15 to about 90 °C, still more typically about 25 °C to about 70 °C.

By aqueous monomer dispersion we mean water or a mixture of water and surfactant, initiator, defoaming agent, or a suitable buffer in cases where pH needs to be kept in a particular range.

The recyclable template particle or core, that may be a solid particle or a hollow particle, is then coated with a shell material to generate a core/shell particle. To generate a silica treatment comprising a coating, layer or shell, at least one water-based silica precursor is used.

The water-based silica precursor in step (c) is sodium silicate, potassium silicate or pre-formed silicic acid; more typically sodium silicate or potassium silicate; still more typically sodium silicate.

The concentration of water-based silica precursor is about 0.005 wt% to about 20 wt%, more typically about 0.005 wt% to about 15 wt%, based on the total weight of the dispersion. Typically, the suspension of recyclable template particles in water is treated with water-based silica precursor, which results in silica deposition of the recyclable particles, generating core/shell particles.

The pH is maintained at about 2 to about 10, more typically about 5 to about 9, to form a silica layer on the recyclable template particle and the reaction times are held between about 1 to about 24 hours, more typically about 1.5 to about 18 hours, still more typically about 2 to about 12 hours. This results in the deposition of a silica treatment comprising a coating, layer or shell on the recyclable template particle or core. The reaction is kept at temperatures between about 25 to about 100 °C, more typically between about 40 and about 90 °C, still more typically between about 50 and about 80 °C.

The core/shell particles are removed from the aqueous solution by centrifugation or filtration, more typically by centrifugation.

Depending on the nature of the recyclable template particle, the recyclable template particle that constitutes the core is then recycled either through thermal depolymerization, or acid- or base hydrolysis.

Typically, core materials made out of poly-(a-methylstyrene), PMMA, various polyamides, as well as styrene are depolymerized at increased temperatures, with the temperatures of depolymerization varying with the polymer used. Some suitable temperature ranges include about 250 to about 450 °C, more typically about 275 to about 400 °C, still more typically from about 290 to about 325 °C, to generate hollow particles as well as core monomer. For example, poly(methylmethacrylate)@silica core/shell particles can be heated above around about 300 °C to generate methyl methacrylate monomer and hollow silica particles. Further, poly(a-methylstyrene)@silica can be heated to about above 60 °C to generate hollow silica particles and α-methylstyrene monomer.

Alternatively, acid- or base-labile core materials can be hydrolyzed instead of thermally depolymerized to generate hollow particles with the possibility of monomer recycling. Polymers such as Delrin® (polyacetal), poly(lactic acid), as well as other polyesters can be depolymerized through acid hydrolysis. For example, treating polyacetal@silica with acid should generate hollow silica as well as aldehyde monomer that can be recycled in template particle synthesis. Similarly, polyesters or polyamides from core/shell particles can be recycled in the same fashion to generate diacid/dialcohol (diacid/diamine) monomer couples as well as hydroxylic or amino acids as monomers (like in the case of polylactic acid, for example).

These depolymerization methods allow for hollow particle formation, as well as, being non-destructive toward core monomers, allowing for template material recycling.

### Applications:

These inorganic hollow particle dispersions are useful as hiding or opacifying agents in coating and molding compositions. They are also useful as drug delivery systems in the pharmaceutical and medical industries; in food, personal care and cosmetics; and agriculture.

### EXAMPLES

### Example 1. Preparation of PMMA recyclable template particle

To a three-necked 250mL round bottom flask with 100.0 mL water was added methyl methacrylate (9.5g, 94.89mmol), 2-(methacryloxy)-ethyltrimethylammonium chloride (0.125g of 80% aqueous solution, mmol), ethylene glycol dimethacrylate (0.4g, mmol), and AlBA (0.1g, mmol). Trimethoxysilyl propyl methacrylate (0.5 g 2.01 mmol) was then added. The mixture was degassed by purging N₂ for 10min, and then heated to 70 °C under nitrogen overnight, to generate a white slurry of PMMA particles.

### Example 2. Preparation of PMMA/silica core/shell particle

To a 100ml three-necked round bottom flask, equipped with an overhead stirrer and a reflux condenser was added 25mL of PMMA suspension from Example 1, and the pH of the mixture was adjusted to 9 with addition of aqueous NaOH. To this mixture was added sodium silicate (4.72g of 26.5wt% aqueous solution, diluted to 10mL with water), and HCl (10mL of 1.40M solution), using two syringe pumps simultaneously, to maintain the pH in the 8-9 range. The rate of addition was 2mL/h. After the addition, the temperature of the reaction was increased to 80 °C, and the mixture was left stirring overnight. After that time, the mixture was cooled down, and the solids isolated by centrifugation, and washed with water and ethanol, to generate a while solid (2.73g), whose TEM confirmed the core/shell structure.

### Example 3. Hollow particle synthesis from PPMA/silica core/shell particles

500mg of the sample from Example 2 was placed in a tube furnace and heated to 500 C at a 1 °C/min rate, and kept at 500 C for ten hours. Upon cooling, 233mg of white solid was obtained, whose TEM images confirmed the hollow structure.

### Example 4. Template recycling

Upon washing, of the material from Example 2, the core/shell particles are placed in a 50mL round bottom flask, and the flask is placed inside a bulb-to-bulb distillation apparatus. The material is heated to 300 °C under nitrogen, and the distillate is collected in the cooled (-20 °C) receiving adapter, to capture the released MMA monomer. The hollow particle material remains in the distillation flask.

## Claims

1. A process for making hollow inorganic particles comprising:
(a) providing a recyclable template particle in an aqueous dispersion, wherein the recyclable template particle is prepared from an organic monomer;
(b) coating the recyclable template particle with a water-based silica precursor;
(c) maintaining the pH at about 2 to about 10 to form core/shell particles comprising a silica treatment on the recyclable template particle;
(d) removing the core/shell particles;
(e) removing the recyclable template particle from the core/shell particles by depolymerization through thermal depolymerization, acid hydrolysis or base hydrolysis to form a hollow silica particle, and
(f) recycling the organic monomer after depolymerization of the recyclable template particle.

2. The process of claim 1 wherein the water-based silica precursor is sodium silicate, potassium silicate or pre-formed silicic acid.

3. The process of claim 2 wherein the water-based silica precursor is sodium silicate or potassium silicate.

4. The process of claim 1, 2 or 3 wherein the recyclable template particle comprises poly-(methylmethacrylate), poly-(alphamethylstyrene), polyamide or polystyrene.

5. The process of claim 4 wherein the recyclable template particle comprising a silica treatment is heated to promote depolymerization.

6. The process of claim 5 wherein the thermal depolymerization occurs at temperatures of about 50 °C to about 600 °C, preferably at temperatures of about 250°C to about 450°C, more preferably at temperatures of about 275°C to about 400°C, still more preferably at temperatures of about 290°C to about 325°C.

7. The process of claim 1, 2 or 3 wherein the recyclable template particle comprises polyamide, polyacetal, poly(lactic acid) or polyester.

8. The process of claim 7 wherein the recyclable template particle comprising a silica treatment is treated with acid.

9. The process of any one of claims 1 to 8 wherein the recyclable template particle is a solid particle.

10. The process of any one of claims 1 to 9 wherein the recyclable template particle is a hollow particle.

11. The process of any one of claims 1 to 10 wherein the particle formed is about 100 nm to about 900 nm in size.

12. The process of any one of claims 1 to 11 wherein the organic monomer comprises styrene, methyl methacrylate, α-methylstyrene, lactic acid, formaldehyde, hydroxyacids, aminoacids, diacids and dialcohols, or diacids and diamines.

13. The process of any one of claims 1 to 12 wherein the pH is maintained at about 5 to about 9.

## Patentansprüche

1. Verfahren zum Herstellen anorganischer Hohlpartikel, umfassend:
(a) Bereitstellen eines recycelbaren Vorlagepartikels in einer wässrigen Dispersion, wobei das recycelbare Vorlagepartikel aus einem organischen Monomer hergestellt wird;
(b) Beschichten des recycelbaren Vorlagepartikels mit einem Siliciumdioxidvorläufer auf Wasserbasis;
(c) Halten des pH-Werts bei etwa 2 bis etwa 10, um Kern-/Mantelpartikel zu bilden, die eine Siliciumdioxidbehandlung auf dem recycelbaren Vorlagepartikel umfassen;
(d) Entfernen der Kern-/Mantelpartikel;
(e) Entfernen der recycelbaren Vorlagepartikel von den Kern-/Mantelpartikeln durch Depolymerisation durch thermische Depolymerisation, saure Hydrolyse oder basische Hydrolyse, um ein Siliciumdioxidhohlpartikel zu bilden, und
(f) Recyceln des organischen Monomers nach der Depolymerisation des recycelbaren Vorlagepartikels.

2. Verfahren nach Anspruch 1, wobei der Siliciumdioxidvorläufer auf Wasserbasis Natriumsilicat, Kaliumsilicat oder vorgebildete Kieselsäure ist.

3. Verfahren nach Anspruch 2, wobei der Siliciumdioxidvorläufer auf Wasserbasis Natriumsilicat oder Kaliumsilicat ist.

4. Verfahren nach Anspruch 1, 2 oder 3, wobei das recycelbare Vorlagepartikel Poly(methylmethacrylat), Poly(alphamethylstyrol), Polyamid oder Polystyrol umfasst.

5. Verfahren nach Anspruch 4, wobei das recycelbare Vorlagepartikel, das eine Siliciumdioxidbehandlung umfasst, erhitzt wird, um die Depolymerisation zu unterstützen.

6. Verfahren nach Anspruch 5, wobei die thermische Depolymerisation bei Temperaturen von etwa 50 °C bis etwa 600 °C, bevorzugt bei Temperaturen von etwa 250 °C bis etwa 450 °C, noch bevorzugter bei Temperaturen von etwa 275 °C bis etwa 400 °C, selbst noch bevorzugter bei Temperaturen von etwa 290 °C bis etwa 325 °C stattfindet.

7. Verfahren nach Anspruch 1, 2 oder 3, wobei das recycelbare Vorlagepartikel Polyamid, Polyacetal, Poly(milchsäure) oder Polyester umfasst.

8. Verfahren nach Anspruch 7, wobei das recycelbare Vorlagepartikel, das eine Siliciumdioxidbehandlung umfasst, mit Säure behandelt wird.

9. Verfahren nach irgendeinem der Ansprüche 1 bis 8, wobei das recycelbare Vorlagepartikel ein festes Partikel ist.

10. Verfahren nach irgendeinem der Ansprüche 1 bis 9, wobei das recycelbare Vorlagepartikel ein Hohlpartikel ist.

11. Verfahren nach irgendeinem der Ansprüche 1 bis 10, wobei das gebildete Partikel etwa 100 nm bis etwa 900 nm in der Größe beträgt.

12. Verfahren nach irgendeinem der Ansprüche 1 bis 11, wobei das organische Monomer Styrol, Methylmethacrylat, α-Methylstyrol, Milchsäure, Formaldehyd, Hydroxysäuren, Aminosäuren, Disäuren und Dialkohole oder Disäuren und Diamine umfasst.

13. Verfahren nach irgendeinem der Ansprüche 1 bis 12, wobei der pH-Wert bei etwa 5 bis etwa 9 gehalten wird.

## Revendications

1. Procédé de fabrication de particules inorganiques creuses comprenant:
(a) la fourniture d'une particule modèle recyclable dans une dispersion aqueuse, la particule modèle recyclable étant préparée à partir d'un monomère organique;
(b) le revêtement de la particule modèle recyclable avec un précurseur de silice à base d'eau;
(c) le maintien du pH à environ 2 à environ 10 pour former des particules type coeur/écorce comprenant un traitement de silice sur la particule modèle recyclable;
(d) l'élimination des particules type coeur/écorce;
(e) l'élimination de la particule modèle recyclable des particules type coeur/écorce par dépolymérisation par une dépolymérisation thermique, hydrolyse acide ou hydrolyse basique pour former une particule de silice creuse, et
(f) le recyclage du monomère organique après dépolymérisation de la particule modèle recyclable.

2. Procédé selon la revendication 1, le précurseur de silice à base d'eau étant le silicate de sodium, le silicate de potassium ou l'acide silicique préformé.

3. Procédé selon la revendication 2, le précurseur de silice à base d'eau étant le silicate de sodium ou le silicate de potassium.

4. Procédé selon les revendications 1, 2 ou 3, la particule modèle recyclable comprenant du poly-(méthacrylate de méthyle), du poly-(alphaméthylstyrène), du polyamide ou du polystyrène.

5. Procédé selon la revendication 4, la particule modèle recyclable comprenant un traitement de silice étant chauffée pour favoriser la dépolymérisation.

6. Procédé selon la revendication 5, la dépolymérisation thermique apparaissant à des températures d'environ 50°C à environ 600°C, préférablement à des températures d'environ 250°C à environ 450°C, plus préférablement à des températures d'environ 275°C à environ 400°C, encore plus préférablement à des températures d'environ 290°C à environ 325°C.

7. Procédé selon les revendications 1, 2 ou 3, la particule modèle recyclable comprenant du polyamide, du polyacétal, du poly(acide lactique) ou du polyester.

8. Procédé selon la revendication 7, la particule modèle recyclable comprenant un traitement de silice étant traitée avec de l'acide.

9. Procédé selon l'une quelconque des revendications 1 à 8, la particule modèle recyclable étant une particule solide.

10. Procédé selon l'une quelconque des revendications 1 à 9, la particule modèle recyclable étant une particule creuse.

11. Procédé selon l'une quelconque des revendications 1 à 10, la particule formée étant d'environ 100 nm à environ 900 nm de taille.

12. Procédé selon l'une quelconque des revendications 1 à 11, le monomère organique comprenant du styrène, du méthacrylate de méthyle, de l'α-méthylstyrène, de l'acide lactique, du formaldéhyde, des hydroxyacides, des acides aminés, des diacides et des dialcools, ou des diacides et des diamines.

13. Procédé selon l'une quelconque des revendications 1 à 12, le pH étant maintenu entre environ 5 et environ 9.
